# EUROPEAN PATENT APPLICATION

(11) **EP 4 647 049 A1**
(43) Date of publication of application: **12.11.2025**
(21) Application number: 23914618.6
(22) Date of filing: 30.12.2023
(51) Int. Cl.: A61F 5/44, A61F 5/451, A61F 5/452, A61G 9/00

(54) **NURSING MACHINE**

(30) Priority: 05.01.2023 CN 202310013418; 28.07.2023 CN 202322020389 U
(71) Applicant: Suzhou Alton Electrical & Mechanical Industry Co., Ltd., Suzhou, Jiangsu 215211 (CN)
(72) Inventor: LU, Weidong, Chicago Illinois (US); SONG, Qiang, Suzhou, Jiangsu 215211 (CN); LI, Wei, Suzhou, Jiangsu 215211 (CN); LI, Zhouen, Suzhou, Jiangsu 215211 (CN); LEI, Dengke, Suzhou, Jiangsu 215211 (CN)
(74) Representative: Ricker, Mathias
(86) International application number: PCT/CN2023/143713
(87) International publication number: WO 2024/146487

(57) **Abstract**

Disclosed is a nursing machine, including an excrement collection assembly for being worn on a human body to receive a human excrement and a negative pressure source configured for suctioning the human excrement. The excrement collection assembly includes an air cushion for contact with the human body. The nursing machine further includes at least one control chip, an air pipeline, an air pump configured for inflating the air cushion, an air pressure sensor configured for monitoring air pressure, and a relief valve configured for relieving pressure. The air pump inflates the air cushion through the air pipeline. The air pressure sensor is configured for directly or indirectly monitoring an air pressure in the air cushion. The relief valve is configured for directly or indirectly relieving a pressure in the air cushion. The air pump and the relief valve are controlled by the at least one control chip. The user experience of the air cushion of the excrement collection assembly of the nursing machine is improved.

## Description

The disclosure claims the priority to Chinese Patent Application No. 202310013418.5, filed to the China National Intellectual Property Administration (CNIPA) on January 5, 2023, and Chinese Patent Application No. 202322020389.3, filed to the China National Intellectual Property Administration (CNIPA) on July 28, 2023, which are hereby incorporated by reference in their entireties.

### Technical Field

The disclosure relates to a nursing machine, and in particular to a nursing machine including an excrement collection assembly with an air cushion.

### Background

A nursing machine is configured for suctioning and cleaning a human excrement, and usually includes a main unit, an excrement collection assembly, a sewage suction pipeline and a water supply pipeline connected between the excrement collection assembly and the main unit, and the like, the main unit includes a negative pressure source for suctioning the human excrement, a wastewater tank for storing the human excrement suctioned by the negative pressure source, and a water pump; the excrement collection assembly includes a wrapping pants apparatus and an excrement collector; the excrement collector includes an excrement recess for accommodating the human excrement; and under the suction of the negative pressure source, the excrement excreted from a human body into the excrement recess are suctioned into the wastewater tank through the suction pipeline for storage. The Chinese patent CN205659042U published on October 26, 2016, discloses an automatic excrement treatment apparatus (i.e., a nursing machine), which includes an air cushion for contact with a human body and an inflation device for inflating the air cushion. However, the air cushion of the excrement collection assembly of the nursing machine has a simple and crude configuration for inflation and deflation, cannot bring about good user experience and needs improvement.

### Summary

The disclosure provides an improved nursing machine, to improve a user experience of an air cushion of an excrement collection assembly of the nursing machine.

Some embodiments of the disclosure provide a nursing machine, including an excrement collection assembly for being worn on a human body to receive a human excrement and a negative pressure source configured for suctioning the human excrement. The excrement collection assembly includes an air cushion for contact with the human body. The nursing machine further includes at least one control chip, an air pipeline, an air pump configured for inflating the air cushion, an air pressure sensor configured for monitoring air pressure, and a relief valve configured for relieving pressure. The air pump inflates the air cushion through the air pipeline. The air pressure sensor is configured for directly or indirectly monitoring an air pressure in the air cushion. The relief valve is configured for directly or indirectly relieving a pressure in the air cushion. The air pump and the relief valve are controlled by the at least one control chip.

In some embodiments, the air pressure sensor is electrically connected to the at least one control chip, and a pressure value monitored by the air pressure sensor is outputted to the at least one control chip. The excrement collection assembly includes a wrapping pants apparatus and an excrement collector. The excrement collector includes an excrement recess configured for receiving the human excrement. The air cushion is part of the wrapping pants apparatus. A first frame opening, for the human body to excrete, is provided and surrounded by the air cushion. The wrapping pants apparatus further includes a connecting bracket configured for connecting the wrapping pants apparatus with the excrement collector. A second frame opening, for the human body to excrete, is provided and surrounded by the connecting bracket. In a state that the connecting bracket and the excrement collector are butted together, the connecting bracket and the excrement collector fit together. The connecting bracket is located on an outer side of a crotch of the wrapping pants apparatus. The air cushion is located on an inner side of the crotch of the wrapping pants apparatus.

In some embodiments, the air cushion is in a form of an annular washer. The wrapping pants apparatus further includes a waist cushion, the waist cushion is communicated with the air cushion, and the waist cushion is inflated and deflated through the air cushion.

In some embodiments, the nursing machine further includes a main unit. The air pump, the air pressure sensor, the relief valve, and the negative pressure source are respectively part of the main unit and are located inside the main unit. The air pressure sensor indirectly monitors the air pressure in the air cushion by monitoring an air pressure in the air pipeline. The relief valve is configured for relieving a pressure in the air pipeline to indirectly relieve the pressure in the air cushion.

In some embodiments, the at least one control chip includes a first control chip. The air pressure sensor, the air pump, and the relief valve are respectively electrically connected to the first control chip. The air pump and the relief valve are respectively controlled by the first control chip.

In some embodiments, during inflation using the air pump, a time taken by the air pump to complete air intaking and air discharging once is a first cycle. The first control chip is set with a second cycle. During inflation using the air pump, the air pressure sensor is able to monitor to obtain a plurality of air pressure monitoring values in each second cycle, and the first control chip uses a minimum value of all the air pressure monitoring values obtained by monitoring by the air pressure sensor in the second cycle as the air pressure value in the air cushion in the second cycle. The second cycle is not shorter than the first cycle.

In some embodiments, an air pressure value monitored by the air pressure sensor is outputted to the first control chip. During inflation using the air pump, in a state that the air pressure monitored by the air pressure sensor reaches a first predetermined value, the air pump stops inflating based on control of the first control chip. In a state that the air pressure monitored by the air pressure sensor drops to a second predetermined value, based on control of the first control chip, the air pump starts inflating again until the air pressure monitored by the air pressure sensor reaches the first predetermined value.

In some embodiments, the at least one control chip further includes a main control chip, and the first control chip is electrically connected to the main control chip and is able to receive a control instruction from the main control chip.

In some embodiments, the nursing machine further includes a heater, an air supply apparatus, and a drying pipeline. The at least one control chip further includes a second control chip. The excrement collection assembly includes a wrapping pants apparatus and an excrement collector. The excrement collector includes an excrement recess configured for receiving the human excrement. The air cushion is part of the wrapping pants apparatus. A drying airflow is formed under a heating of the heater and a driving of air by the air supply apparatus. The drying airflow is transmitted through the drying pipeline and supplied to the excrement collector. The second control chip is electrically connected to the main control chip and is able to receive a control instruction from the main control chip. The heater and the air supply apparatus are respectively electrically connected to the second control chip and controlled by the second control chip.

In some embodiments, the at least one control chip further includes a third control chip. The third control chip is able to receive a control instruction from the main control chip. The negative pressure source is electrically connected to the third control chip and controlled by the third control chip. The negative pressure source is able to perform suction under control of the third control chip.

For the disclosure, by means of the air pressure sensor, the at least one control chip, and the like, more accurate and controllable inflation of the air cushion using the air pump is facilitated; and by means of the relief valve and the at least one control chip, more convenient and controllable pressure relief of the air cushion is facilitated. In summary, the user experience of the air cushion of the excrement collection assembly of the nursing machine is improved.

### Brief Description of the Drawings

Fig. 1 is an architecture block diagram of a nursing machine;
Fig. 2 is a three-dimensional diagram of a main unit (connected with pipelines, and the like) of a nursing machine;
Fig. 3 is another three-dimensional diagram of a main unit of a nursing machine (with part of a shell of the main unit removed);
Fig. 4 is a three-dimensional diagram of an excrement collection assembly connected with pipelines and the like of a nursing machine from one perspective;
Fig. 5 is a three-dimensional diagram of an excrement collection assembly connected with pipelines and the like of a nursing machine from another perspective;
Fig. 6 is a three-dimensional diagram of an excrement collection assembly of a nursing machine;
Fig. 7 is an exploded view of an excrement collection assembly of a nursing machine;
Fig. 8 is a three-dimensional diagram of a wrapping pants apparatus of a nursing machine;
Fig. 9 is another three-dimensional diagram of a wrapping pants apparatus of a nursing machine;
Fig. 10 is a schematic diagram of a front side of a wrapping pants main portion, an air cushion, and a waist cushion of a wrapping pants apparatus of a nursing machine in an unfolded state;
Fig. 11 is a schematic diagram of a back side of a wrapping pants main portion, an air cushion, and a waist cushion of a wrapping pants apparatus of a nursing machine in an unfolded state;
Fig. 12 is a schematic diagram of a front side of an air cushion and a waist cushion of a wrapping pants apparatus of a nursing machine in an unfolded state (with a second communicating pipeline connected between the air cushion and the waist cushion);
Fig. 13 is a schematic diagram of a back side of an air cushion and a waist cushion of a wrapping pants apparatus of a nursing machine in an unfolded state (with a second communicating pipeline connected between the air cushion and the waist cushion);
Fig. 14 is a three-dimensional diagram of an excrement collector of a nursing machine;
Fig. 15 is another three-dimensional diagram of an excrement collector of a nursing machine (with a stuck seat removed);
Fig. 16 is a horizontal cross-sectional view of an excrement collector and a connecting bracket of a wrapping pants apparatus of a nursing machine being butted together;
Fig. 17 is a three-dimensional diagram of a first coil module, a circuit board module, a first cable, and an electrical connector of a nursing machine being connected together;
Fig. 18 is a three-dimensional diagram of a first connecting socket, a first main hose, a second main hose, a second connecting socket, and the like of a nursing machine being connected together; and
Fig. 19 is a three-dimensional schematic diagram of Fig. 18 with a first lower cover of the first connecting socket and a second lower cover of the second connecting socket removed.

### Detailed Description of the Embodiments

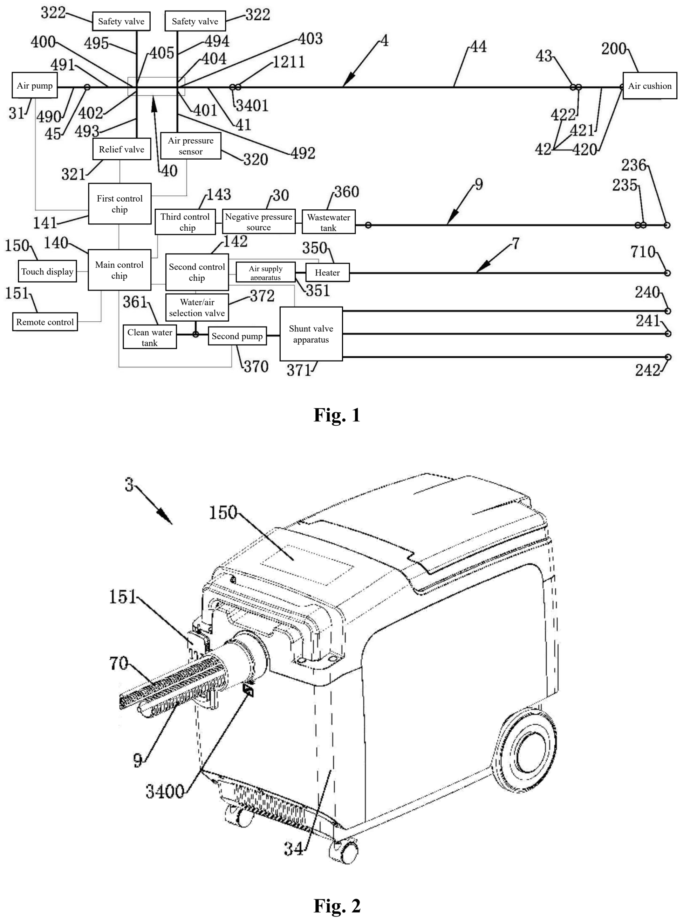

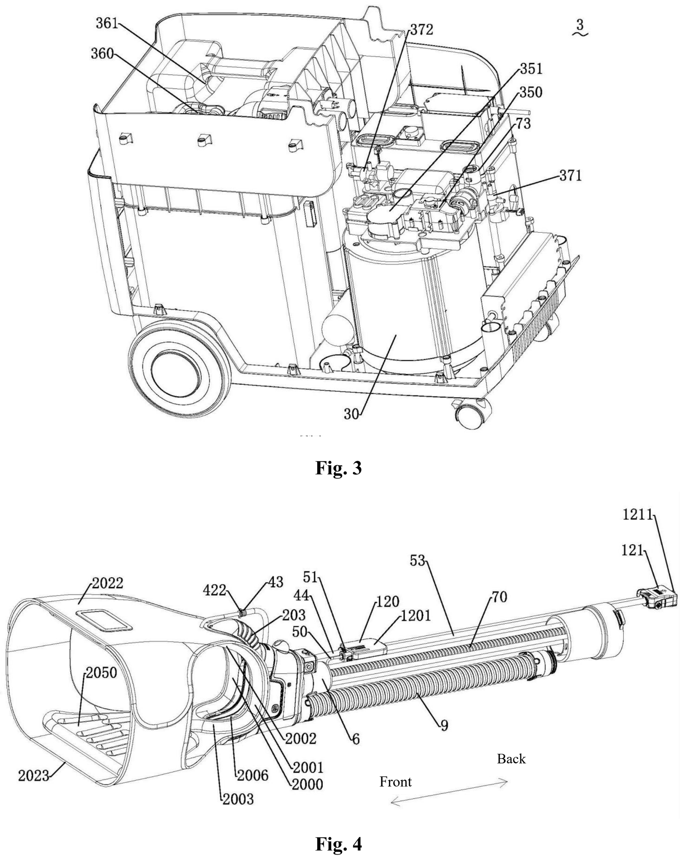

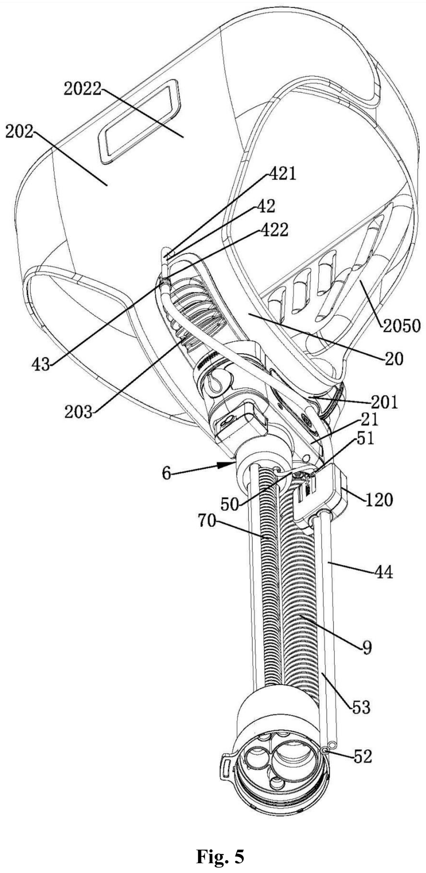

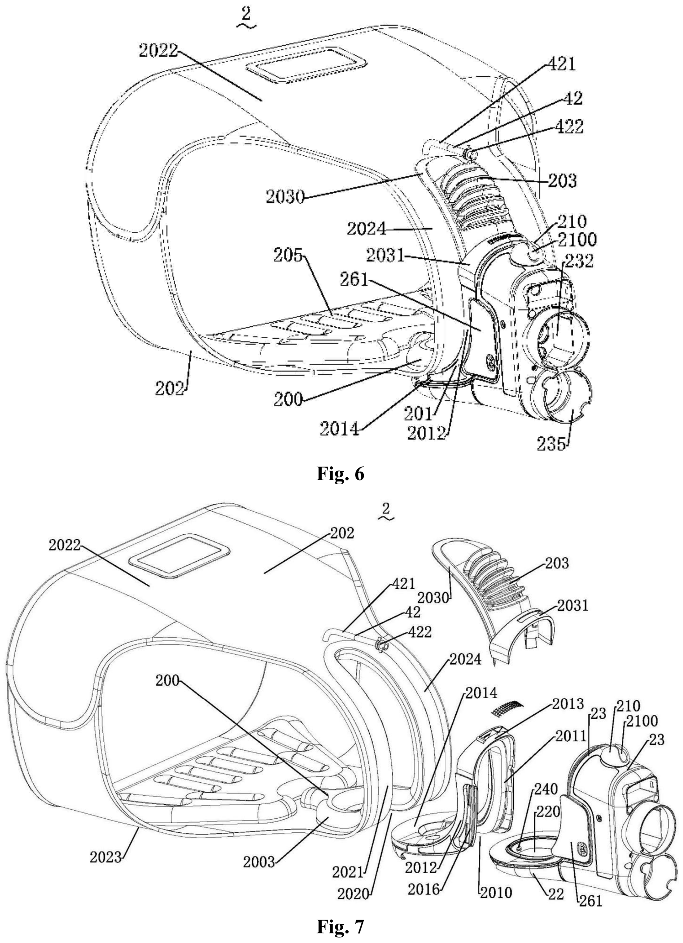

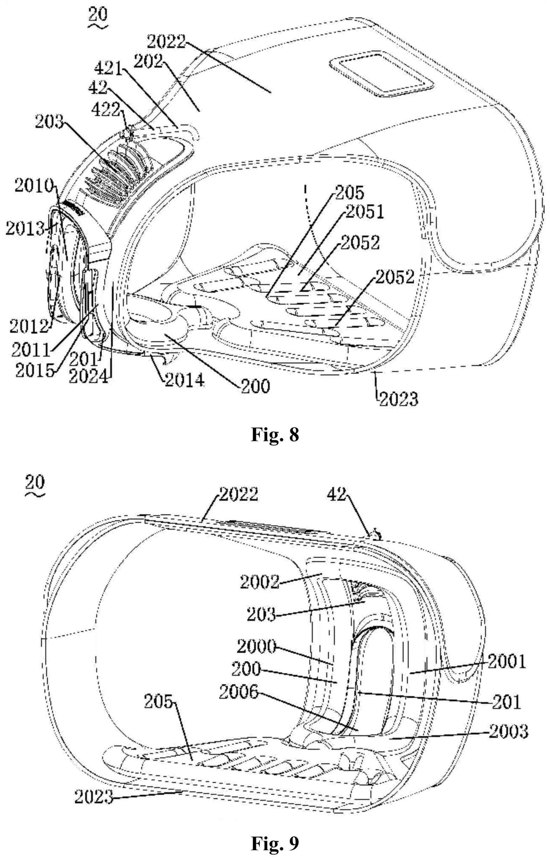

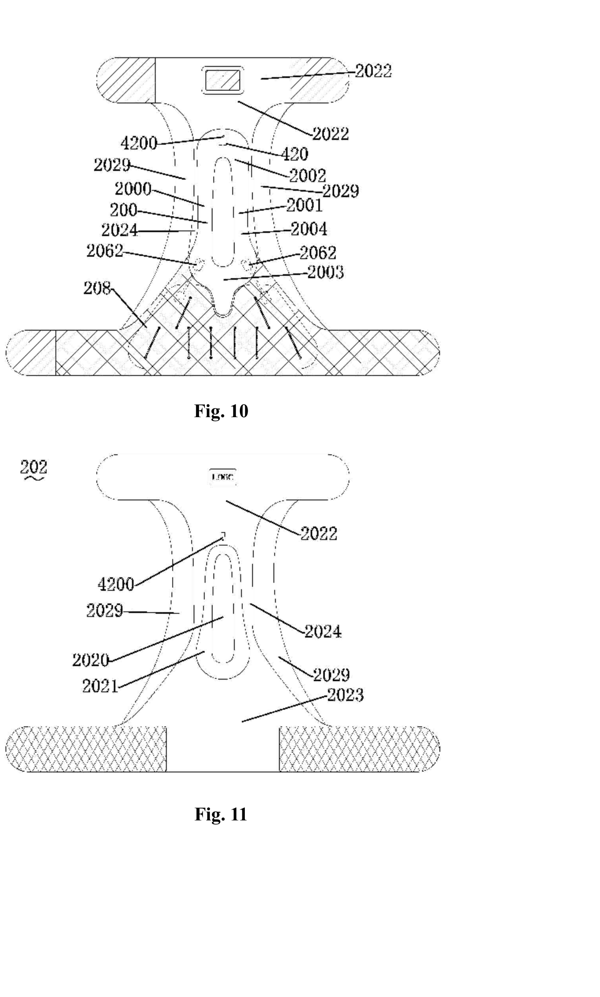

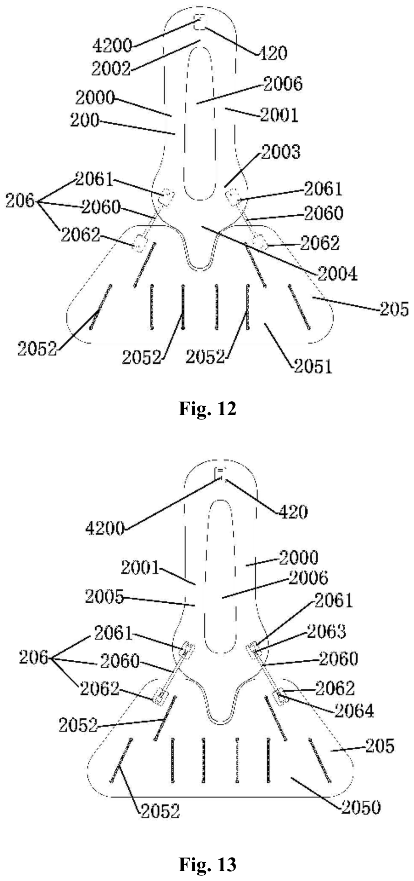

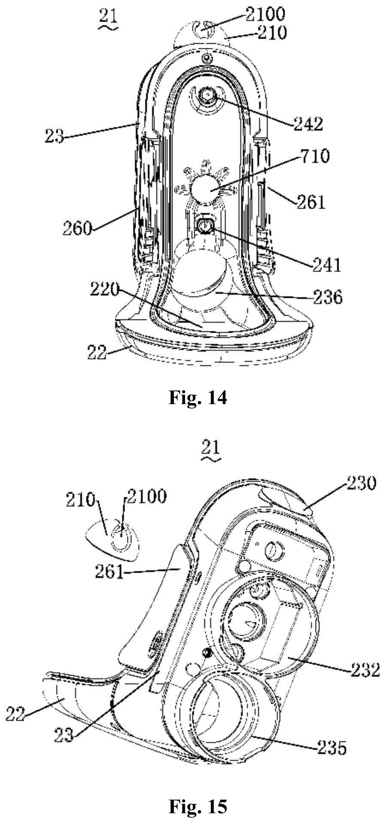

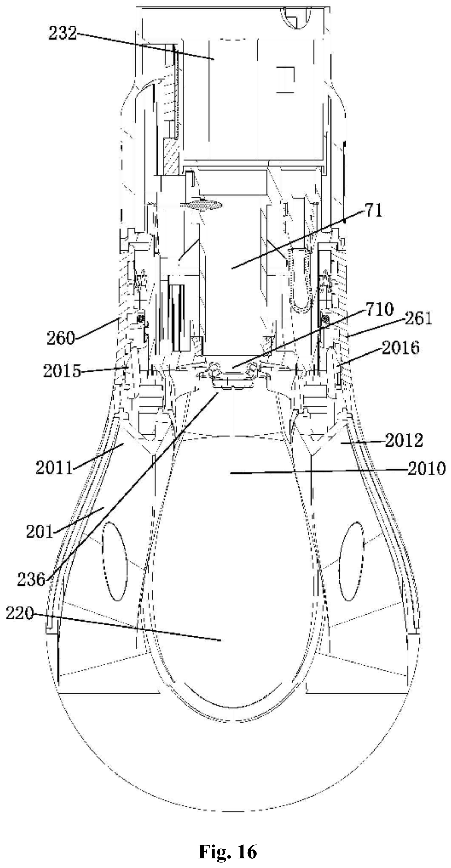

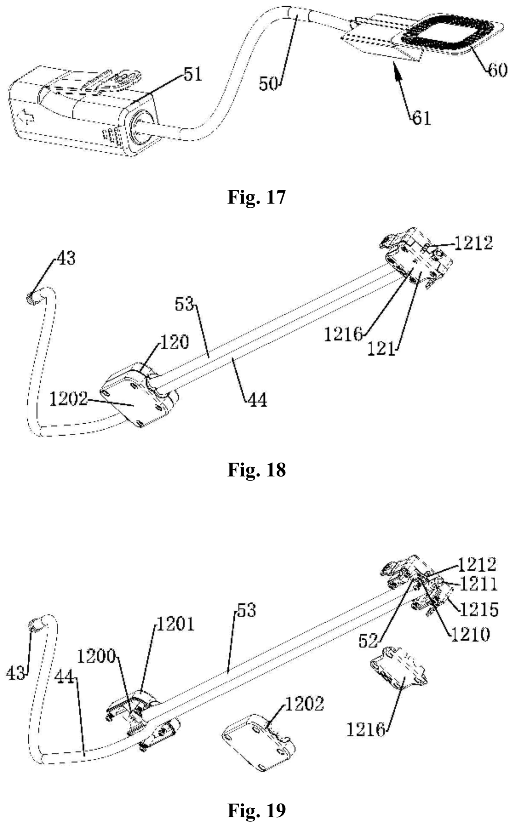

As shown in Figs. 1-19, a nursing machine of the disclosure includes a negative pressure source 30, an excrement collection assembly 2, at least one control chip, an air pipeline 4, an air pump 31, an air pressure sensor 320 configured for monitoring air pressure, a relief valve 321 configured for relieving pressure, and a safety valve 322, there is at least one safety valve 322. The negative pressure source 30 is configured for suctioning a human excrement. The excrement collection assembly 2 is configured for being worn on a human body to receive the human excrement. The excrement collection assembly 2 includes an air cushion 200. The air cushion 200 is configured for contact with the human body to seal a contact part between the excrement collection assembly 2 and the human body, to prevent leakage of excrement from between the excrement collection assembly 2 and the human body. The air pipeline 4 is connected to the air cushion 200. The air pump 31 is configured for inflating the air cushion 200. Further, the air pump 31 inflates the air cushion 200 by the air pipeline 4. The air pressure sensor 320 is configured for directly or indirectly monitoring an air pressure in the air cushion 200. The air pressure sensor 320 is electrically connected to the at least one control chip. A pressure value monitored by the air pressure sensor 320 is outputted to the at least one control chip. The relief valve 321 is configured for directly or indirectly relieving a pressure in the air cushion 200. The at least one safety valve 322 is connected to the air pipeline 4. The negative pressure source 30, the air pump 31 and the relief valve 321 are controlled by the at least one control chip. Further, the at least one control chip includes a first control chip 141. The air pressure sensor 320, the air pump 31, and the relief valve 321 are respectively electrically connected to the first control chip 141. The air pressure value monitored by the air pressure sensor 320 is outputted to the first control chip 141. The air pump 31 and the relief valve 321 are respectively controlled by the first control chip 141, and are able to respectively be opened and closed under control of the first control chip 141. By means of the air pressure sensor 320 and the at least one control chip, more accurate and controllable inflation of the air cushion 200 using the air pump 31 is facilitated; and by means of the relief valve 321 and the at least one control chip, more convenient and controllable pressure relief of the air cushion 200 is facilitated.

The safety valve 322 is configured for preventing the air pressure in the air pipeline 4 from being too high, thereby indirectly preventing the air pressure in the air cushion 200 from being too high. The safety valve 322 is a mechanically opened and closed valve, which does not consume electricity and is not controlled by the at least one control chip, thus being not affected by electronic failures and having high reliability. On the other hand, the safety valve 322 is configured for limiting an upper limit of the air pressure in the air pipeline 4, and indirectly, the safety valve 322 is configured for limiting an upper limit of the air pressure in the air cushion 200. By means of the safety valve 322, safety is able to be improved, and the air cushion 200 is prevented from bursting due to excessive air pressure in case of continuous excessive inflation when the nursing machine has an electronic failure. The safety valve 322 has an opening threshold and a reset value. The opening threshold is equivalent to the upper limit of the air pressure in the air cushion 200. When the corresponding air pressure reaches the opening threshold, the safety valve 322 opens. When the corresponding air pressure drops to or below the reset value, the safety valve 322 returns to be closed. The air pressure in the air pipeline 4 needs to rise to the opening threshold again when the safety valve 322 opens the next time. Based on the reset value, the safety valve 322 is able to return to be closed more promptly without the need for complete deflation, thereby avoiding waste caused by the air pipeline 4 being excessively deflated by the safety valve 322, and ensuring that the safety valve 322 is closed during inflation the next time. In the embodiment, the safety valve 322 has an opening threshold of 20 KPa (kilopascals), and a reset value of 8 KPa. Namely, when the air pressure in the air pipeline 4 reaches 20 KPa, the safety valve 322 will open and release air in the air pipeline 4. When the air pressure in the air pipeline 4 drops to 8 KPa, the safety valve 322 will return to be closed. After the safety valve 322 opens, the air is released at a high rate, and the air pressure in the air pipeline 4 is able to be reduced to the reset value in a very short time. Therefore, the safety valve 322 also returns to be closed in a very short time after opening. When the air pressure in the air pipeline 4 reaches the opening threshold of the safety valve 322, the safety valve 322 opens under the air pressure in the air pipeline 4 to release the air in the air pipeline 4, thereby preventing the air cushion 200 from compressing the human body or bursting due to excessive air pressure in the air pipeline 4 and air cushion 200, and ensuring high safety. The disclosure does not improve the safety valve 322 itself, and the safety valve 322 is selected from existing products on the market. Similarly, the disclosure does not improve the relief valve 321 itself, and the relief valve 321 is selected from existing products. The relief valve 321 is a controlled valve. After the excrement of a patient is treated, based on control of the at least one control chip, the relief valve 321 is opened to empty the air pipeline 4 and the air cushion 200, so that the air cushion 200 becomes deflated and the pressure on the human body is relived, so that the human body feels comfortable. Afterwards, the relief valve 321 is closed under control of the at least one control chip. The next time the excrement is treated by the nursing machine, the at least one control chip controls the air pump 31 to inflate the air cushion 200 again.

The nursing machine further includes a main unit 3. The air pipeline 4 is able to fluidly connect the main unit 3 with the excrement collection assembly 2. The air pump 31, the air pressure sensor 320, the relief valve 321, the safety valve 322, and the negative pressure source 30 are respectively part of the main unit 3 and are located inside the main unit 3. The at least one control chip is located inside the main unit 3. The negative pressure source 30 includes a motor and an impeller. The impeller is driven by the motor to rotate to produce a suction airflow for suctioning the excrement. The negative pressure source 30 is also a vacuum pump, and the like.

The air pipeline 4 includes an N-way pipe 40, and the N-way pipe is located inside the main unit 3. The N-way pipe 40 is communicated with the air cushion 200 (the N-way pipe 40 is communicated with the air cushion 200 indirectly through an air pipe). The air pump 31, the air pressure sensor 320, the relief valve 321, and the safety valve 322 are respectively connected to the air pipeline 4 through the N-way pipe 40, thus achieving efficient and convenient connection. The N-way pipe 40 includes a first end 400, a second end 401, and a third end 402 that are intercommunicated. The air pump 31 is correspondingly connected to the first end 400, the air pressure sensor 320 is correspondingly connected to the second end 401, and the relief valve 321 is correspondingly connected to the third end 402. The air pipeline 4 further includes an adapter pipe 41, and the adapter pipe 41 is located inside the main unit 3. The N-way pipe 40 further includes a fourth end 403, a fifth end 404, and a sixth end 405. The first end 400, the second end 401, the third end 402, the fourth end 403, the fifth end 404, and the sixth end 405 are intercommunicated. The adapter pipe 41 is correspondingly connected to the fourth end 403. One safety valve 322 is correspondingly connected to the fifth end 404, and the other safety valve 322 is correspondingly connected to the sixth end 405.

The air pipeline 4 further includes a one-way valve 45. The one-way valve 45 is connected between the first end 400 and the air pump 31 and configured for the air pump 31 to output air to the air cushion 300 in a one-way manner. The air pump 31 is not able to thoroughly avoid air leakage. With the presence of the one-way valve 45, after the air pump 31 stops inflating, the air in the air cushion 200 will not flow back in an opposite direction to the air pump 31 by the air pipeline 4, thereby avoiding leakage. For the convenience of connection, the air pump 31 is connected to the one-way valve 45 by a first pipe section 490, and the one-way valve 45 is connected to the first end 400 by a second pipe section 491. Therefore, the air pump 31 is indirectly connected to the first end 400 by the first pipe section 490, the one-way valve 45, and the second pipe section 491 sequentially. The air pressure sensor 320, the relief valve 321, one safety valve 322, and the other safety valve 322 are respectively connected to the corresponding second end 401, third end 402, fifth end 404, and sixth end 405 by corresponding pipe sections (492, 493, 494, and 495). The first pipe section 490, the second pipe section 491, and the pipe sections (492, 493, 494, and 495) are each a small section of hose.

The excrement collection assembly 2 includes a wrapping pants apparatus 20 and an excrement collector 21. The excrement collector 21 is able to be indirectly worn on the human body by means of the wrapping pants apparatus 201. The excrement collector 21 includes an excrement recess 220 configured for receiving the human excrement. The air cushion 200 is part of the wrapping pants apparatus 20. A first frame opening 2006, for the human body to excrete, is provided and surrounded by the air cushion 200. The wrapping pants apparatus 20 further includes a connecting bracket 201 configured for connecting the wrapping pants apparatus 20 with the excrement collector 21. A second frame opening 2010, for the human body to excrete, is provided and surrounded by the connecting bracket 201. The connecting bracket 201 is able to be made of hard plastic and is in a frame shape. When the connecting bracket 201 and the excrement collector 21 are butted together, the connecting bracket 201 and the excrement collector 21 fit together, and the second frame opening 2010 is butted to the excrement recess 220. The connecting bracket 201 is located on an outer side of a crotch of the wrapping pants apparatus 20. The air cushion 200 is located on an inner side of the crotch of the wrapping pants apparatus 20. The air cushion 200 includes a left section 2000, a right section 2001, a first C-shaped section 2002, and a second C-shaped section 2003. The first C-shaped section 2002 is arranged above the second C-shaped section 2003. The first C-shaped section 2002, the left section 2000, the second C-shaped section 2003, and the right section 2001 are sequentially connected to form a closed loop to form the first frame opening 2006. The connecting bracket 201 includes a left connecting portion 2011, a right connecting portion 2012, a first C-shaped portion 2013, and a second C-shaped portion 2014. The first C-shaped portion 2013, the left connecting portion 2011, the second C-shaped portion 2014, and the right connecting portion 2012 are sequentially connected to form a closed loop to form the second frame opening 2010. The second C-shaped portion 2014 corresponds to the second C-shaped section 2003, the left connecting portion 2011 corresponds to the left section 2000, and the right connecting portion 2012 corresponds to the right section 2001.

The wrapping pants apparatus 20 further includes a wrapping pants main portion 202 and a urine hood 203. The connecting bracket 201 is located on an outer side of the wrapping pants main portion 202. The air cushion 200 is located on an inner side of the wrapping pants main portion 202. The wrapping pants main portion 202 includes a crotch opening 2020, and the crotch opening 2020 is arranged between the first frame opening 2006 and the second frame opening 2010. The first frame opening 2006, the crotch opening 2020, and the second frame opening 2010 are superposed together for the human body to excrete. When the human body excretes, the excrement is able to enter the excrement recess through the first frame opening, the crotch opening, and the second frame opening that are superposed together. The urine hood 203 includes a first C-shaped edge portion 2030 and a second C-shaped edge portion 2031. The second C-shaped edge portion 2031 is correspondingly attached to the first C-shaped portion 2013. The first C-shaped edge portion 2030, the left connecting portion 2011, the second C-shaped portion 2014, and the right connecting portion 2012 are attached to the wrapping pants main portion 202. The first C-shaped edge portion 2030, the left connecting portion 2011, the second C-shaped portion 2014, and the right connecting portion 2012 together form a loop extending along an edge of the crotch opening 2020. The air cushion 200 extends along the edge of the crotch opening 2020 to form a loop. The first C-shaped section 2002 corresponds to the first C-shaped edge portion 2030. The urine hood 203 and the connecting bracket 201 provide support for a back side of the air cushion 200, to maintain a shape of the air cushion 200. The urine hood 203 is made of a flexible material, for example, a silicone material. The main material of the wrapping pants main portion 202 is able to be white foamed styrene butadiene rubber, with a yellow nylon mesh attached to a surface of the white foamed styrene butadiene rubber.

The wrapping pants main portion 202 further includes an annular adhesive layer 2021. The annular adhesive layer 2021 is configured for adhering to the urine hood 203 and the connecting bracket 201. The annular adhesive layer 2021 is located on a back side of the wrapping pants main portion 202. The annular adhesive layer 2021 is sewn onto the back side of the wrapping pants main portion 202. The annular adhesive layer 2021 surrounds the crotch opening 2020 of the wrapping pants main portion 202. The annular adhesive layer 2021 extends along the edge of the crotch opening 2020 to form a loop. The first C-shaped edge portion 2030, the left connecting portion 2011, the second C-shaped portion 2014, and the right connecting portion 2012 are adhered to the annular adhesive layer 2021, thereby attaching to the wrapping pants main portion 202. The second C-shaped edge portion 2031 and the first C-shaped portion 2013 are adhered together. The annular adhesive layer 2021 is able to be made of genuine leather, synthetic leather, polyvinyl chloride (PVC) material, or the like.

The wrapping pants main portion 202 further includes an upper wrapping sheet 2022, a lower wrapping sheet 2023, a connecting part 2024, and a modesty cloth 2029. The connecting part 2024 connects the upper wrapping sheet 2022 with the lower wrapping sheet 2023. The connecting part 2024 is provided with the crotch opening 2020. The connecting bracket 201 and the air cushion 200 are located on opposite sides of the connecting part 2024. In a state that the connecting bracket 201 and the excrement collector 21 are butted together, the air cushion 200, the connecting part 2024, the connecting bracket 201, and the excrement collector 21 are sequentially distributed from front to back. The modesty cloth 2029 is connected to the connecting part 2024 and arranged on left and right sides of the connecting part 2024. The modesty cloth 2029 allows the wrapping pants apparatus 20 to cover a larger area of the private parts of the human body. The modesty cloth 2029 makes the human body wearing the wrapping pants apparatus 20 less likely to be exposed. The modesty cloth 2029 is able to be made of polyester coated single jersey.

The air pipeline 4 is directly communicated with the first C-shaped section 2002. The wrapping pants apparatus 20 further includes a first communicating pipeline 42, and the first communicating pipeline 42 forms part of the air pipeline 4. The first communicating pipeline 42 is configured for an external communication of the air cushion 200. The first communicating pipeline 42 includes a first connector 420, a first hose 421, and a first quick coupling 422. The first connector 420 includes a first air tap 4200. One end of the first hose 421 is connected to the first air tap 4200. The other end of the first hose 421 is connected to the first quick coupling 422. The first connector 420 is directly communicated with the first C-shaped section 2002. The air cushion 200, the first connector 420, the first hose 421, and the first quick coupling 422 are fluidly communicated sequentially. Such a design facilitates quick external connection of the air cushion 200 by the first communicating pipeline 42 with the first quick coupling 422. One end of the first hose 421 is sleeved on the first air tap 4200. The first connector 420 is located at a tail end of the air pipeline 4. The first connector 420 is arranged on a back side of the first C-shaped section 2002 and maintains direct communication with the first C-shaped section 2002 at all times. The first connector 420 is at least partially located in the first C-shaped section 2002. The first air tap 4200 is located on a top side of the wrapping pants main portion 202. The first air tap 4200 extends out of the air cushion 200 and passes through the wrapping pants main portion 202 to extend to the top side of the wrapping pants main portion 202. The first air tap 4200 is located on a front side of the urine hood 203. The first hose 421 is able to be connected to the first air tap 4200 conveniently.

The air cushion 200 is in a form of an annular washer. Further, the air cushion 200 is in the form of an annular washer corresponding to the crotch opening 2020. The wrapping pants apparatus 20 further includes a waist cushion 205. The waist cushion 205 is communicated with the air cushion 200. The waist cushion 205 is inflated and deflated through the air cushion 200. During inflation using the air pump 31, the airflow outputted flows through the air cushion 200 to the waist cushion 205, to inflate the waist cushion 205. When the waist cushion 205 is deflated, the air released from the waist cushion 205 flows through the air cushion 200. The wrapping pants apparatus 20 further includes a second communicating pipeline 206, and there is at least one second communicating pipeline 206. The second communicating pipeline 206 is configured for fluid communication between the waist cushion 205 and the air cushion 200. The second communicating pipeline 206 includes a second hose 2060, a second connector 2061, and a third connector 2062. The second hose 2060 communicates the second connector 2061 with the third connector 2062. The second connector 2061 is directly communicated with the air cushion 200. The second connector 2061 includes a second air tap 2063. The third connector 2062 is directly communicated with the waist cushion 205. The third connector 2062 includes a third air tap 2064. One end of the second hose 2060 is connected to the second air tap 2063. The other end of the second hose 2060 is connected to the third air tap 2064. Such a design effectively achieves fluid communication between the waist cushion 205 and the air cushion 200. The one end of the second hose 2060 is sleeved on the second air tap 2063, and the other end of the second hose 2060 is sleeved on the third air tap 2064. During inflation, air flowing through the air cushion 200 further flows through the second connector 2061, the second hose 2060, and the third connector 2062 sequentially to inflate the waist cushion 205. The waist cushion 205 is arranged on the lower wrapping sheet 2023, and is configured for padding a bottom side of the waist of the human body (i.e., the waist cushion 205 is configured for padding the lower back of the human body), thereby effectively preventing the waist of the human body wearing the wrapping pants apparatus 20 from being suspended. The second connector 2061 is at least partially located in the second C-shaped section 2003. The second air tap 2063 is located at a bottom of the second C-shaped section 2003. The one end of the second hose 2060 extends to the bottom of the second C-shaped section 2003, to be butted to the second air tap 2063. The third connector 2062 is at least partially located in the waist cushion 205. The third air tap 2064 is located at a bottom of the waist cushion 205. The other end of the second hose 2060 extends to the bottom of the waist cushion 205, to be butted to the third air tap 2064. Such arrangement is also able to prevent the second air tap 2063 and the third air tap 2064 from coming into contact with the human body and causing discomfort. A number of the second communicating pipeline 206 is two, and the two second communicating pipelines 206 are arranged at left and right respectively. The second hose 2060 is able to be a silicone hose.

The waist cushion 205 includes an inner sheet 2050, an outer sheet 2051, and a plurality of hot-pressed belts 2052. The plurality of hot-pressed belts 2052 are arranged on the inner sheet 2050 and the outer sheet 2051. The plurality of hot-pressed belts 2052 are formed by hot pressing, specifically by hot pressing the inner sheet 2050 and the outer sheet 2051. An inner space of the waist cushion 205 is formed between the inner sheet 2050 and the outer sheet 2051. The plurality of hot-pressed belts 2052 pull the inner sheet 2050 and the outer sheet 2051 after the waist cushion 205 is inflated, to limit a distance between the inner sheet 2050 and the outer sheet 2051, maintain the waist cushion 205 to be in a flat shape, and prevent the waist cushion 205 from expanding excessively and even into a ball shape. The inner sheet 2050 is directly in contact with the wrapping pants main portion 202. After the wrapping pants apparatus 20 is worn on the human body, the outer sheet 2051 is between the waist of the human body and the inner sheet 2050. In the embodiment, each of the plurality of hot-pressed belts 2052 is formed by hot pressing part of the inner sheet 2050 with part of the outer sheet 2051 together. The wrapping pants apparatus 20 further includes a covering cloth 208, and the covering cloth 208 covers the outer sheet 2051 to prevent the outer sheet 2051 from directly contacting the waist of the human body, and provides protection for the waist cushion 205 and the outer sheet 2051. The covering cloth 208 is able to be breathable mesh fabric.

The air pipeline 4 further includes a second quick coupling 43 and a first main hose 44. The second quick coupling 43 is detachably butted to the first quick coupling 422 correspondingly. One end of the first main hose 44 is connected to the second quick coupling 43 correspondingly. Based on the first quick coupling 422 and the second quick coupling 43, the air cushion 200 is able to be conveniently and quickly connected to the air pipeline for inflation. The first quick coupling 422 and the second quick coupling 43 are able to be butted together by threaded connection. One end of the adapter pipe 41 is connected to the N-way pipe 40. The N-way pipe 40, the adapter pipe 41, and the first main hose 44 are fluidly communicated sequentially. An airflow outputted by the air pump 31 flows through the N-way pipe 40, the adapter pipe 41, and the first main hose 44 sequentially. Further, the airflow outputted by the air pump 31 flows through the N-way pipe 40, the adapter pipe 41, the first main hose 44, and the first communicating pipeline 42 sequentially.

The excrement collector 21 includes a stuck seat 210. The stuck seat 210 is fixedly arranged at a top of the excrement collector 21. The stuck seat 210 includes a stuck slot 2100, and part of the air pipeline 4 is stuck in the stuck slot 2100. The stuck seat 210 plays a role in putting the air pipeline 4 in place, so that the air pipeline 4 is less likely to get messy. In the embodiment, part of the first main hose 44 is stuck into the stuck slot 2100. Of course, the first hose 421 is also made longer, so that part of the first hose 421 is designed to be stuck into the stuck slot 2100. The excrement collector 21 includes a chassis portion 22 and a rear seat portion 23. The chassis portion 22 extends forward from the rear seat portion 23. The rear seat portion 23 extends upward and is higher than the chassis portion 22. The excrement recess 220 is concavely arranged at the chassis portion 22. The stuck seat 210 is adhered fixedly to a top of the rear seat portion 23. The rear seat portion 23 includes a protruding portion 230 corresponding to the stuck seat 210. The protruding portion 230 is arranged at the top of the rear seat portion 23 and is to be adhered fixedly to the stuck seat 210. The protruding portion 230 is disc-shaped.

The nursing machine further includes a first cable 50, an electrical connector 51, a second cable 52, a second main hose 53 corresponding to the second cable 52, a first connecting socket 120, and a second connecting socket 121. The first cable 50 and the second cable 52 are configured for supplying power from the main unit 3 to the excrement collector 21. The second main hose 53 and the first main hose 44 are in parallel and are integrally formed. One end of the second main hose 53 is located inside the first connecting socket 120. The first main hose 44 runs through the first connecting socket 120. The first connecting socket 120 facilitates assembly of the first main hose 44 with the second main hose 53. The other end of the second main hose 53 is located inside the second connecting socket 121. One end of the first main hose 44 is located inside the second connecting socket 121. The second connecting socket 121 also facilitates assembly of the first main hose 44 with the second main hose 53. The second main hose 53 is sleeved on the second cable 52 (i.e., the second cable 52 is threaded through the second main hose 53). The second main hose 53 provides protection for the second cable 52 to prevent the second cable 52 from being damaged due to pulling or squeezing. One end of the second cable 52 is located inside the first connecting socket 120 and extends from the one end of the second main hose 53. The other end of the second cable 52 is located inside the second connecting socket 121 and extends from the other end of the second main hose 53. The first connecting socket 120 includes a first electrical connecting module 1200, a first upper cover 1201, and a first lower cover 1202. One end of the second main hose 53 and one end of the second cable 52 are able to be pressed together by an aluminum ring, and the aluminum ring is clamped between the first upper cover 1201 and the first lower cover 1202. The second connecting socket 121 includes a second upper cover 1215 and a second lower cover 1216. The other end of the second main hose 53 and the other end of the second cable 52 are able to be pressed together by another aluminum ring, and the aluminum ring is clamped between the second upper cover 1215 and the second lower cover 1216. The first electrical connecting module 1200 includes a terminal plate and a plurality of first metal terminals, configured for electrical connection with the second cable 52 and the electrical connector 51. The second cable 52 extends into the first connecting socket 120 and is connected to the first electrical connecting module 1200. The electrical connector 51 is able to be plugged into the first connecting socket 120. The first electrical connecting module 1200 is electrically connected to the electrical connector 51 plugged into the first connecting socket 120. In a state that the electrical connector 51 is plugged into the first connecting socket 120, the second cable 52, the first electrical connecting module 1200, the electrical connector 51, and the first cable 50 are electrically connected sequentially. The second connecting socket 121 includes a second electrical connecting module 1210 and an air joint 1211. The air joint 1211 is connected to the first main hose 44 and forms part of the air pipeline 4. The second electrical connecting module 1210 includes a terminal plate and a plurality of second metal terminals 1212, configured for electrical connection with the second cable 52 and the main unit 3. The second connecting socket 121 is able to be plugged into the main unit 3. The main unit 3 includes a second mating interface portion 3400 corresponding to the second connecting socket 121, and the second connecting socket 121 is able to be plugged into the second mating interface portion 3400. In a state that the second connecting socket 121 is plugged into the second mating interface portion 3400, the second electrical connecting module 1210 is electrically connected to the main unit 3 and the air joint 1211 is fluidly communicated with the main unit 3. The second mating interface portion 3400 includes a third electrical connecting module and an air interface 3401. In a state that the second connecting socket 121 is plugged into the second mating interface portion 3400, the second electrical connecting module 1210 is butted to the third electrical connecting module by the plurality of second metal terminals 1212 and thereby is electrically connected to the main unit 3, and the air joint 1211 is butted to the air interface 3401 and thereby is fluidly communicated with the main unit 3. The second mating interface portion 3400 is arranged on a front wall of a shell 34 of the main unit 3. One end of the adapter pipe 41 is correspondingly connected to the fourth end 403, and the other end of the adapter pipe 41 is correspondingly connected to the air interface 3401. The adapter pipe 41 is able to be a silicone hose.

The N-way pipe 40, the adapter pipe 41, the air joint 1211, the first main hose 44, the second quick coupling 43, the first quick coupling 422, the first hose 421, and the first connector 420 are fluidly communicated sequentially. An airflow outputted by the air pump 31 flows through the N-way pipe 40, the adapter pipe 41, the air joint 1211, the first main hose 44, the second quick coupling 43, the first quick coupling 422, the first hose 421, and the first connector 420 sequentially. Further, the first pipe section 490, the one-way valve 45, the second pipe section 491, the N-way pipe 40, the adapter pipe 41, the air interface 3401, the air joint 1211, the first main hose 44, the second quick coupling 43, the first quick coupling 422, the first hose 421, and the first connector 420 are fluid communicated sequentially to form the air pipeline 4. An airflow outputted by the air pump 31 flows through the first pipe section 490, the one-way valve 45, the second pipe section 491, the N-way pipe 40, the adapter pipe 41, the air interface 3401, the air joint 1211, the first main hose 44, the second quick coupling 43, the first quick coupling 422, the first hose 421, and the first connector 420 sequentially, and then enters the air cushion 200 to inflate the air cushion 200.

The left section 2000, the first C-shaped section 2002, and the right section 2001 are connected sequentially. The first connector 420 is arranged on the back side of the first C-shaped section 2002. The urine hood 203 is in a raised state, and in a state that the connecting bracket 201 is butted to the excrement collector 21, the urine hood 203 is located above the excrement recess 220. The first connector 420 is adjacent to the first C-shaped edge portion 2030. The first C-shaped edge portion 2030 and the first C-shaped section 2002 correspond to each other and are located on opposite sides of the wrapping pants main portion 202. The air cushion 200 is sewn onto the wrapping pants main portion 202. The first C-shaped edge portion 2030 is adhered to the wrapping pants main portion 202. Such a design facilitates production of the wrapping pants apparatus 20. The air cushion 200 includes a first TPU layer 2004, a second TPU layer, and a fabric layer 2005. The fabric layer 2005 and the second TPU layer are stacked together. An internal space of the air cushion 200 is formed between the first TPU layer 2004 and the second TPU layer. The first TPU layer 2004 is for contact with the human body. The full name of TPU is thermoplastic polyurethane. The first TPU layer 2004 forms a surface layer of the air cushion 200 for contact with the human body. The TPU layer has the characteristics of slight breathability and waterproofing, is able to provide slight breathability to the air cushion 200, reduce the probability of bedsores in users, and prevent excessive breathability from causing significant air leakage of the air cushion 200. The first TPU layer 2004 is semi-transparent. The fabric layer 2005 and the second TPU layer are directly stacked together to form an inner layer of the air cushion 200. The fabric layer 2005 greatly increases the strength of the inner layer of the air cushion 200, and prevents the air cushion 200 from bursting.

The at least one control chip includes a main control chip 140 and a second control chip 142. The first control chip 141 and the second control chip 142 are respectively electrically connected to the main control chip 140. The first control chip 141 and the second control chip 142 are able to respectively receive control instructions from the main control chip 140 and are also able to output control instructions to the main control chip 140. Under normal conditions, the air pressure in the air pipeline 4 is the same as that in the air cushion 200. The pressure sensor 320 indirectly monitors the air pressure in the air cushion 200 by monitoring the air pressure in the air pipeline 4, and the relief valve 321 is configured for relieving the pressure in the air pipeline 4 to indirectly relieve the pressure in the air cushion 200. During inflation using the air pump 31, a time taken by the air pump 31 to complete air intaking and air discharging once is a first cycle. The at least one control chip (e.g., the first control chip 141) is set with a second cycle. During inflation using the air pump 31, the air pressure sensor 320 is able to monitor to obtain a plurality of air pressure monitoring values in each second cycle, and the at least one control chip (e.g., the first control chip 141) uses a minimum value of all the air pressure monitoring values obtained by monitoring by the air pressure sensor 320 in the second cycle as the air pressure value of the air cushion 200 in the second cycle. The second cycle is not shorter than the first cycle. During inflation using the air pump 31, air is transmitted from the air pump 31 to the air cushion 200 in a certain process. At the moment when the air is outputted from the air pump 31, the air is not able to be fully transmitted from the air pump 31 to the air cushion 200. Therefore, at the moment when the air is outputted from the air pump 31 (at which point the air pressure in the air pipeline 4 is particularly unstable), the air pressure value in the air pipeline 4 measured by the air pressure sensor 320 is not able to effectively reflect the air pressure in the air cushion 200. Using the minimum value of all the air pressure values monitored by the air pressure sensor 320 in the corresponding second cycle as the air pressure value of the air cushion 200 in the second cycle, Distorted air pressure values measured at the moment when the air is outputted by the air pump 31 are able to be filtered out, and the true air pressure in the air cushion 200 in the corresponding second cycle during inflation using the air pump 31 is able to be more accurately reflected, so that the air pressure in the air cushion 200 is able to be more accurately monitored during inflation using the air pump 31. The first cycle reflects the inflating frequency of the air pump 31. Assuming that the inflating frequency of the air pump 31 is n times per second, the first cycle is one nth of a second. The inflating frequency of the air pump 31 being n times per second means that the air pump 31 completes inflation and deflation n times per second. With the maturity of the air pump 31 technology, the inflating frequency of the current air pump 31 is able to be very high, and easily reach thousands of times per second. During inflation using the air pump 31, the air pressure sensor 320 monitors the air pressure in the air pipeline 4 in real time. Therefore, the air pressure sensor 320 is able to monitor to obtain and transmit a plurality of air pressure monitoring values in each second cycle to the at least one control chip (e.g., the first control chip 141). The at least one control chip (e.g., the first control chip 141) makes a choice based on the above rule (i.e., uses the minimum value of all the air pressure monitoring values in the second cycle). In the embodiment, the second cycle is able to be set to be longer, for example, the second cycle is three times the first cycle. When the air pressure value used by the at least one control chip (e.g., the first control chip 141) reaches a predetermined value, the at least one control chip (e.g., the first control chip 141) is able to control the air pump 31 to stop inflating. The air pump 31 is able to be a diaphragm pump.

During inflation using the air pump 31, when the air pressure in the air pipeline 4 reaches a first predetermined value (or when the air pressure monitored by the air pressure sensor 320 reaches the first predetermined value), the air pump 31 stops inflating based on control of the at least one control chip (e.g., the first control chip 141). When the air pressure in the air pipeline 4 drops to a second predetermined value (or when the air pressure monitored by the air pressure sensor 320 drops to the second predetermined value), based on control of the at least one control chip (e.g., the first control chip 141), the air pump 31 starts inflating again until the air pressure in the air pipeline 4 reaches the first predetermined value. Thus, the air pressure in the air cushion 200 is dynamically maintained in a predetermined air pressure range (from the second predetermined value to the first predetermined value), and a relatively constant pressure in the air cushion 200 is achieved. The disclosure does not improve the air pressure sensor 320 itself, and the air pressure sensor 320 is selected from existing products on the market. The air pressure sensor 320 is able to continuously output the monitored air pressure values in real time for use by the at least one control chip (e.g., the first control chip 141). In the embodiment, during inflation using the air pump 31, when the air pressure value used by the at least one control chip (e.g., the first control chip 141) reaches the first predetermined value, the air pressure in the air pipeline 4 is considered to reach the first predetermined value, and also the air pressure in the air cushion 200 is considered to reach the first predetermined value. After the air pump 31 stops inflating, the air pressures in the air pipeline 4 and the air cushion 200 are very stable. In this case, the air pressure value monitored by the air pressure sensor 320 in real time is able to match the actual air pressure values in the air pipeline 4 and the air cushion 200. Therefore, after the air pump 31 stops inflating, when the air pressure value in the air pipeline 4 monitored by the air pressure sensor 320 in real time reaches the second predetermined value, the air pressure in the air pipeline 4 is considered to drop to the second predetermined value, and also the air pressure in the air cushion 200 is considered to drop to the second predetermined value. The nursing machine is able to provide a plurality of inflation air pressure ranges for users to choose from. For example, the first predetermined value is set as 10.5 KPa, and the second predetermined value is set as 9.5 KPa, so that a first inflation air pressure range is 9.5 KPa to 10.5 KPa. In another case, the first predetermined value is set as 8.5 KPa, and the second predetermined value is set as 7.5 KPa, so that a second inflation air pressure range is 7.5 KPa to 8.5 KPa.

The nursing machine further includes a touch display 150. The touch display 150 is able to be used for controlling the air pump 31 and/or the relief valve 321. The touch display 150 is electrically connected to the main control chip 140. The main control chip 140 is able to transmit a corresponding instruction to the first control chip 141 based on an instruction inputted by the touch display 150, for the first control chip 141 to control the air pump 31 and/or the relief valve 321. In the embodiment, the touch display 150 is part of the main unit 3 and is arranged on a top wall of the shell 34 of the main unit 3. The nursing machine further includes a remote controller 151, and the remote controller 151 is able to be used for controlling the air pump 31 and/or the relief valve 321. The remote controller 151 is able to be a wireless remote controller or a wired remote controller. The main control chip 140 is able to transmit a corresponding instruction to the first control chip 141 based on an instruction inputted by the remote controller 151, for the first control chip 141 to control the air pump 31 and/or the relief valve 321.

The nursing machine further includes a heater 350, an air supply apparatus 351, and a drying pipeline 7. The drying pipeline 7 is connected to the excrement collector 21. A drying airflow is formed under a heating of the heater 350 and a driving of air by the air supply apparatus 351. The drying airflow is transmitted through the drying pipeline 7 and supplied to the excrement collector 21 for drying the private parts of the human body. The heater 350 and the air supply apparatus 351 are respectively electrically connected to the second control chip 142 and controlled by the at least one control chip (e.g., the second control chip 142) to open and close. The heater 350 is able to use a positive temperature coefficient (PTC) heater, in which a plurality of PTC ceramic heating elements are arranged. The air supply apparatus 351 is able to be a fan.

The at least one control chip further includes a third control chip 143. The negative pressure source 30 is electrically connected to the third control chip 143 and controlled by the third control chip 143 to open and close. The third control chip 143 is able to receive a control instruction from the main control chip 140 and is also able to output a control instruction to the main control chip 140. The main control chip 140, the first control chip 141, the second control chip 142, and the third control chip 143 are part of the main unit 3 and are located in the shell 34 of the main unit 3. The main control chip 140 is able to play a role in centrally scheduling and coordinating work, facilitate the smoother work of the whole nursing machine system and the control chips thereof, and avoid conflict and delay of work tasks. Each control chip is responsible for the corresponding control work to ensure efficient operation of the system. The nursing machine includes a first operating mode and a second operating mode. In the first operating mode, the negative pressure source 30 performs suction under control of the at least one control chip (e.g., the third control chip 143), and produces negative pressure at the excrement recess 220, and air flows through the air supply apparatus 351, the heater 350, and the drying pipeline 7 to the excrement recess 220 of the excrement collector 21. Thus, air is supplied to the excrement recess 220, and excrement collected in the excrement recess 220 is able to be sufficiently suctioned. Therefore, the drying pipeline 7 is able to be used as an air supply pipeline. It is common knowledge that air will flow towards negative pressure. The suction of the negative pressure source 30 produces negative pressure in the excrement recess 220, causing air to flow into the excrement collector 21 through the air supply apparatus 351, the heater 350, and the drying pipeline 7. In the second operating mode, the negative pressure source 30 stops suction and the heater 350 and the air supply apparatus 351 work (i.e., the heater 350 heats up and the air supply apparatus 351 operates) under control of the at least one control chip (e.g. the second control chip 142), to form the drying airflow in the drying pipeline 7. In the first operating mode, the air supply apparatus 351 and the heater 350 stops working or does not stop working. The nursing machine further includes a docking assembly 6. The docking assembly 6 includes a first coil module 60 configured for supplying power for the excrement collector 21, and a circuit board module 61. The first coil module 60 and the circuit board module 61 are located inside the docking assembly 6. The circuit board module 61 is electrically connected between the first coil module 60 and the first cable 50. The first coil module 60 is able to be coupled with a second coil module in the excrement collector 21 for wireless power supply and wireless communication. The circuit board module 61 is electrically connected to the first coil module 60 by an electrical wire. The excrement collector 21 further includes a first air pipeline and a first mating interface portion 232. The first mating interface portion 232 is located at a rear end of the first air pipeline 71 and is configured for abutting to the docking assembly 6. The first air pipeline 71 includes an air outlet 710, and the air outlet 710 opens forward and is located on an upper side of the excrement recess 220. The air outlet 710 forms a tail end port of the drying pipeline 7. The drying pipeline 7 further includes a first air hose 70 and a second air pipeline. The second air pipeline is arranged in the main unit 3. The heater 350 and the air supply apparatus 351 are part of the main unit 3 and are located in the shell 34 of the main unit 3. The second air pipeline includes a first hard pipe 73 and a second air hose. The first hard pipe 73, the second air hose, the first air hose 70, and the first air pipeline 71 are communicated sequentially to form the drying pipeline 7. The drying pipeline 7 is located downstream of the heater 350, and the air supply apparatus 351 is located upstream of the heater 350. An airflow driven by the air supply apparatus 351 is heated by the heater 350 to form the drying airflow that flows through the drying pipeline 7 to the excrement collector 21.

The nursing machine further includes a sewage suction pipeline 9, and the sewage suction pipeline 9 is configured for connecting the main unit 3 with the excrement collector 21. The negative pressure source 30 is able to suction the human excrement by the sewage suction pipeline 9. Further, the negative pressure source 30 is able to suction the excrement from the excrement recess 220 by the sewage suction pipeline 9. The main unit 3 further includes a wastewater tank 360 for accommodating the excrement suctioned by the negative pressure source 30. The excrement collector 21 further includes a sewage discharging interface 235 and a sewage discharging port 236. The sewage discharging interface 235 is butted to one end of the sewage suction pipeline 9. The sewage discharging port 236 is directly communicated with the excrement recess 220 and configured for discharging the excrement from the excrement recess 220. Taking the direction in which the excrement collector 21 and the connecting bracket 201 are butted together as a front to back direction, the excrement collector 21 is able to be abutted forward to the connecting bracket 201, to be abutted to the wrapping pants apparatus 20. The excrement collector 21 further includes a left buckle member 260 and a right buckle member 261. The left buckle member 260 and the right buckle member 261 are located on left and right sides of the excrement collector 21. The connecting bracket 201 further includes a left mating portion 2015 to be fastened with the left buckle member 260 and a right mating portion 2016 to be fastened with the right buckle member 261. The left mating portion 2015 and the right mating portion 2016 are located on left and right sides of the connecting bracket 201. The left mating portion 2015 is correspondingly arranged on the left connecting portion 2011. The right mating portion 2016 is correspondingly arranged on the right connecting portion 2012. In a state that the connecting bracket 201 is butted to the excrement collector 21, the left buckle member 260 is fastened to the left mating portion 2015 and the right buckle member 261 is fastened to the right mating portion 2016, thereby locking the excrement collector 21 and the connecting bracket 201 together, and abutting the excrement collector 21 to the wrapping pants apparatus 20. The left buckle member 260 includes at least one left fastening protrusion to be fastened with the left mating portion 2015. The left mating portion 2015 is able to be a mating groove and/or a fastening protrusion. The right buckle member 261 includes at least one right fastening protrusion to be fastened with the right mating portion 2016. As the excrement collector 21 is inserted forward into the connecting bracket 201 of the wrapping pants apparatus 201, the at least one left fastening protrusion is fastened with the left mating portion 2015, and the at least one right fastening protrusion is fastened with the right mating portion 2016.

The air pump 31 is able to be regarded as a first pump. The nursing machine further includes a second pump 370, a shunt valve apparatus 371, and a clean water tank 361. The clean water tank 361 is configured for providing clean water. The shunt valve apparatus 371 is configured for switching water circuits to selectively deliver water from the clean water tank 361 to a first nozzle 240, a second nozzle 241, or a third nozzle 242 by a corresponding water circuit. The second pump 370 is electrically connected to the main control chip 140, and is able to be controlled by the at least one control chip (e.g., the main control chip 140) to open and close. The shunt valve apparatus 371 is able to selectively communicate a first water pipeline corresponding to the first nozzle 240, a second water pipeline 8 corresponding to the second nozzle 241, or a third water pipeline 82 corresponding to the third nozzle 242 under control of the at least one control chip (e.g., the second control chip 142) to switch the water circuits. The nursing machine further includes a water/air selection valve 372. The water/air selection valve 372 is controlled by the at least one control chip (e.g., the second control chip 142), and is electrically connected to the second control chip 142. The water/air selection valve 372 is located upstream of the second pump 370, and is able to select to supply water or air to the second pump 370 under control of the at least one control chip (e.g., the second control chip 142).

## Claims

1. A nursing machine, comprising an excrement collection assembly for being worn on a human body to receive a human excrement, and a negative pressure source configured for suctioning the human excrement, the excrement collection assembly comprising an air cushion for contact with the human body, wherein the nursing machine further comprises at least one control chip, an air pipeline, an air pump configured for inflating the air cushion, an air pressure sensor configured for monitoring air pressure, and a relief valve configured for relieving pressure, the air pump inflates the air cushion through the air pipeline, the air pressure sensor is configured for directly or indirectly monitoring an air pressure in the air cushion, the relief valve is configured for directly or indirectly relieving a pressure in the air cushion, and the air pump and the relief valve are controlled by the at least one control chip.

2. The nursing machine according to claim 1, wherein the air pressure sensor is electrically connected to the at least one control chip; a pressure value monitored by the air pressure sensor is outputted to the at least one control chip; the excrement collection assembly comprises a wrapping pants apparatus and an excrement collector; the excrement collector comprises an excrement recess configured for receiving the human excrement; the air cushion is part of the wrapping pants apparatus; a first frame opening, for the human body to excrete, is provided and surrounded by the air cushion; the wrapping pants apparatus further comprises a connecting bracket configured for connecting the wrapping pants apparatus with the excrement collector; a second frame opening, for the human body to excrete, is provided and surrounded by the connecting bracket; in a state that the connecting bracket and the excrement collector are butted together, the connecting bracket and the excrement collector fit together; the connecting bracket is located on an outer side of a crotch of the wrapping pants apparatus; and the air cushion is located on an inner side of the crotch of the wrapping pants apparatus.

3. The nursing machine according to claim 2, wherein the air cushion comprises a first C-shaped section, a left section, and a right section; the connecting bracket comprises a first C-shaped portion, a second C-shaped portion, a left connecting portion, and a right connecting portion; the first C-shaped portion, the left connecting portion, the second C-shaped portion, and the right connecting portion are sequentially connected to form a closed loop, thereby are enclosed to form the first frame opening; the wrapping pants apparatus further comprises a wrapping pants main portion and a urine hood; the connecting bracket is located on an outer side of the wrapping pants main portion; the air cushion is located on an inner side of the wrapping pants main portion; the wrapping pants main portion comprises a crotch opening; the first frame opening, the crotch opening, and the second frame opening are superposed together for the human body to excrete; the urine hood comprises a first C-shaped edge portion and a second C-shaped edge portion; the second C-shaped edge portion is correspondingly attached to the first C-shaped portion; the first C-shaped edge portion, the left connecting portion, the second C-shaped portion, and the right connecting portion are attached to the wrapping pants main portion; and the first C-shaped edge portion, the left connecting portion, the second C-shaped portion, and the right connecting portion together form a loop extending along an edge of the crotch opening.

4. The nursing machine according to claim 2, wherein the wrapping pants apparatus further comprises a wrapping pants main portion; the connecting bracket is located on an outer side of the wrapping pants main portion; the air cushion is located on an inner side of the wrapping pants main portion; the wrapping pants main portion is provided with a crotch opening and an annular adhesive layer; the first frame opening, the crotch opening, and the second frame opening are superposed together; the annular adhesive layer is configured for adhering to the urine hood and the connecting bracket; the annular adhesive layer is located on a back side of the wrapping pants main portion; the annular adhesive layer is sewn onto the back side of the wrapping pants main portion; the annular adhesive layer extends along an edge of the crotch opening to form a loop; the urine hood comprises a first C-shaped edge portion; the connecting bracket comprises a second C-shaped portion, a left connecting portion, and a right connecting portion; the first C-shaped edge portion, the left connecting portion, the second C-shaped portion, and the right connecting portion together form a loop extending along the edge of the crotch opening; and the first C-shaped edge portion, the left connecting portion, the second C-shaped portion, and the right connecting portion are adhered to the annular adhesive layer.

5. The nursing machine according to claim 2, wherein the wrapping pants apparatus further comprises a wrapping pants main portion; the wrapping pants main portion comprises an upper wrapping sheet, a lower wrapping sheet, and a connecting part; the connecting part is connected to the upper wrapping sheet and the lower wrapping sheet; the wrapping pants main portion is provided with a crotch opening; the crotch opening is arranged on the connecting part; the connecting bracket and the air cushion are located on opposite sides of the connecting part; the excrement collector is able to be forwardly butted with the connecting bracket; in a state that the connecting bracket and the excrement collector are butted together, the air cushion, the connecting part, the connecting bracket, and the excrement collector are sequentially distributed from front to back.

6. The nursing machine according to claim 1, wherein the excrement collection assembly comprises a wrapping pants apparatus and an excrement collector; the excrement collector comprises an excrement recess configured for receiving the human excrement; the air cushion is part of the wrapping pants apparatus; the air cushion comprises a first C-shaped section, a left section, and a right section; the left section, the first C-shaped section, and the right section are connected sequentially; the wrapping pants apparatus further comprises a first communicating pipeline; the first communicating pipeline forms part of the air pipeline; the first communicating pipeline is configured for an external communication of the air cushion; the first communicating pipeline comprises a first connector, a first hose, and a first quick coupling; the first connector comprises a first air tap; one end of the first hose is connected to the first air tap; the other end of the first hose is connected to the first quick coupling; the first connector is directly communicated with the first C-shaped section; and the air cushion, the first connector, the first hose, and the first quick coupling are fluidly communicated sequentially.

7. The nursing machine according to claim 6, wherein the nursing machine further comprises a safety valve configured for limiting an upper limit of the air pressure in the air cushion, and there is at least one safety valve; the air pipeline further comprises a second quick coupling to be detachably butted to the first quick coupling correspondingly, and a first main hose; the second quick coupling is connected to one end of the first main hose; the nursing machine further comprises a main unit; the air pump, the air pressure sensor, the relief valve, the safety valve, and the negative pressure source are respectively part of the main unit and are located inside the main unit; the air pipeline further comprises an adapter pipe and an N-way pipe; the adapter pipe and the N-way pipe are located inside the main unit; the air pump, the air pressure sensor, the relief valve, and the safety valve are respectively connected to the air pipeline through the N-way pipe; one end of the adapter pipe is connected to the N-way pipe; and an airflow outputted by the air pump flows through the N-way pipe, the adapter pipe, the first main hose, and the first communicating pipeline sequentially.

8. The nursing machine according to claim 6, wherein the wrapping pants apparatus further comprises a wrapping pants main portion and a urine hood; the first connector is arranged on a back side of the first C-shaped section; the first air tap is arranged on a top side of the wrapping pants main portion; the first air tap is arranged on a front side of the urine hood; the urine hood comprises a first C-shaped edge portion; the first connector is adjacent to the first C-shaped edge portion; the first C-shaped edge portion and the first C-shaped section correspond to each other and are located on opposite sides of the wrapping pants main portion; the air cushion is sewn onto the wrapping pants main portion; and the first C-shaped edge portion is adhered to the wrapping pants main portion.

9. The nursing machine according to claim 1, wherein the excrement collection assembly comprises a wrapping pants apparatus and an excrement collector; the excrement collector comprises an excrement recess configured for receiving the human excrement; the air cushion is part of the wrapping pants apparatus; the air cushion is in a form of an annular washer; the wrapping pants apparatus further comprises a waist cushion; the waist cushion is communicated with the air cushion; and the waist cushion is inflated and deflated through the air cushion.

10. The nursing machine according to claim 9, wherein the wrapping pants apparatus further comprises a second communicating pipeline, and there is at least one second communicating pipeline; the second communicating pipeline is configured for fluid communication between the waist cushion and the air cushion; the second communicating pipeline comprises a second hose, a second connector, and a third connector; the second hose communicates the second connector with the third connector; the second connector is directly communicated with the air cushion; the second connector comprises a second air tap; the third connector is directly communicated with the waist cushion; the third connector comprises a third air tap; one end of the second hose is connected to the second air tap; and the other end of the second hose is connected to the third air tap.

11. The nursing machine according to claim 9, wherein the waist cushion comprises an inner sheet, an outer sheet, and a plurality of hot-pressed belts arranged on the inner sheet and the outer sheet; an inner space of the waist cushion is formed between the inner sheet and the outer sheet; the plurality of hot-pressed belts pull the inner sheet and the outer sheet after the waist pad is inflated; the wrapping pants apparatus further comprises a wrapping pants main portion and a covering cloth; the covering cloth covers the outer sheet; the wrapping pants main portion comprises an upper wrapping sheet, a lower wrapping sheet, and a connecting part; the connecting part connects the upper wrapping sheet with the lower wrapping sheet; the connecting part is provided with a crotch opening; and the waist cushion is arranged on the lower wrapping sheet.

12. The nursing machine according to claim 1, wherein the nursing machine further comprises a safety valve, and there is at least one safety valve; the safety valve is connected to the air pipeline; the safety valve does not consume electricity and is not controlled by the at least one control chip; the safety valve is configured for limiting an upper limit of the air pressure in the air cushion; in a state that an air pressure in the air pipeline rises to an opening threshold, the safety valve is opened from a closed state to relieve a pressure in the air pipeline; in a state that the air pressure in the air pipeline drops to or below a reset value, the safety valve returns to the closed state from an open state; the air pressure sensor is electrically connected to the at least one control chip; and a pressure value monitored by the air pressure sensor is outputted to the at least one control chip.

13. The nursing machine according to claim 1, wherein the nursing machine further comprises a main unit; the air pump, the air pressure sensor, the relief valve, and the negative pressure source are respectively part of the main unit and are located inside the main unit; the air pressure sensor indirectly monitors the air pressure in the air cushion by monitoring an air pressure in the air pipeline; and the relief valve is configured for relieving a pressure in the air pipeline to indirectly relieve the pressure in the air cushion.

14. The nursing machine according to claim 13, wherein the nursing machine further comprises a safety valve, and there is at least one safety valve; the safety valve is part of the main unit and is located inside the main unit; the air pipeline comprises an N-way pipe; the N-way pipe is located inside the main unit; the N-way pipe is communicated with the air cushion; and the air pump, the air pressure sensor, the relief valve, and the safety valve are respectively connected to the air pipeline through the N-way pipe.

15. The nursing machine according to claim 14, wherein the N-way pipe comprises a first end, a second end, and a third end; the first end, the second end, and the third end are intercommunicated; the air pipeline is further provided with a one-way valve; the one-way valve is connected between the first end and the air pump and configured for the air pump to output air to the air cushion in a one-way manner; the air pressure sensor is correspondingly connected to the second end; and the relief valve is correspondingly connected to the third end.

16. The nursing machine according to claim 15, wherein the air pipeline further comprises an adapter pipe and a first main hose; the adapter pipe is located inside the main unit; the first main hose is located outside the main unit; the N-way pipe further comprises a fourth end, a fifth end, and a sixth end; the first end, the second end, the third end, the fourth end, the fifth end, and the sixth end are intercommunicated; the adapter pipe is correspondingly connected to the fourth end; one of the at least one safety valve is correspondingly connected to the fifth end; the other of the at least one safety valve is correspondingly connected to the sixth end; and an airflow outputted by the air pump flows through the N-way pipe, the adapter pipe, and the first main hose sequentially.

17. The nursing machine according to claim 1, wherein the excrement collection assembly comprises a wrapping pants apparatus and an excrement collector; the excrement collector comprises an excrement recess configured for receiving the human excrement; the air cushion is part of the wrapping pants apparatus; the excrement collector comprises a stuck seat; the stuck seat is fixedly arranged at a top of the excrement collector; the stuck seat comprises a stuck slot; part of the air pipeline is stuck in the stuck slot; the excrement collector further comprises a chassis portion and a rear seat portion; the chassis portion extends forward from the rear seat portion; the rear seat portion extends upward and is higher than the chassis portion; the excrement recess is concavely arranged at the chassis portion; and the stuck seat is arranged at a top of the rear seat portion.

18. The nursing machine according to claim 1, wherein the air pipeline further comprises a first main hose; the nursing machine further comprises a first cable, an electrical connector, a second cable, a second main hose corresponding to the second cable, a first connecting socket, and a second connecting socket; the first cable is connected to the electrical connector; the electrical connector is able to be plugged into the first connecting socket; the second main hose and the first main hose are in parallel and are integrally formed; one end of the second main hose is located inside the first connecting socket; the other end of the second main hose is located inside the second connecting socket; the first main hose runs through the first connecting socket; one end of the first main hose is located inside the second connecting socket; the second main hose is sleeved on the second cable; one end of the second cable is located inside the first connecting socket and extends from the one end of the second main hose; the other end of the second cable is located inside the second connecting socket and extends from the other end of the second main hose; the nursing machine further comprises a main unit; the air pump, the air pressure sensor, the relief valve, and the negative pressure source are respectively part of the main unit and are located inside the main unit; the main unit further comprises a second mating interface portion corresponding to the second connecting socket; and the second connecting socket is able to be plugged into the second mating interface portion.

19. The nursing machine according to claim 1, wherein the at least one control chip comprises a first control chip; the air pressure sensor, the air pump, and the relief valve are respectively electrically connected to the first control chip; and the air pump and the relief valve are respectively controlled by the first control chip.

20. The nursing machine according to claim 19, wherein the air pressure sensor indirectly monitors the air pressure in the air cushion by monitoring an air pressure in the air pipeline; during inflation using the air pump, a time taken by the air pump to complete air intaking and air discharging once is a first cycle; the first control chip is set with a second cycle; during inflation using the air pump, the air pressure sensor is able to monitor to obtain a plurality of air pressure monitoring values in each second cycle, and the first control chip uses a minimum value of all the air pressure monitoring values obtained by monitoring by the air pressure sensor in the second cycle as the air pressure value in the air cushion in the second cycle; and the second cycle is not shorter than the first cycle.

21. The nursing machine according to claim 19, wherein the air pressure sensor indirectly monitors the air pressure in the air cushion by monitoring an air pressure in the air pipeline; an air pressure value monitored by the air pressure sensor is outputted to the first control chip; during inflation using the air pump, in a state that the air pressure monitored by the air pressure sensor reaches a first predetermined value, the air pump stops inflating based on control of the first control chip; and in a state that the air pressure monitored by the air pressure sensor drops to a second predetermined value, based on control of the first control chip, the air pump starts inflating again until the air pressure monitored by the air pressure sensor reaches the first predetermined value.

22. The nursing machine according to claim 19, wherein the air pressure sensor indirectly monitors the air pressure in the air cushion by monitoring an air pressure in the air pipeline; an air pressure value monitored by the air pressure sensor is outputted to the first control chip; during inflation using the air pump, in a state that the air pressure in the air pipeline reaches a first predetermined value, the air pump stops inflating based on control of the first control chip; and in a state that the air pressure in the air pipeline drops to a second predetermined value, based on control of the first control chip, the air pump starts inflating again until the air pressure in the air pipeline reaches the first predetermined value.

23. The nursing machine according to claim 19, wherein the at least one control chip further comprises a main control chip, and the first control chip is electrically connected to the main control chip and is able to receive a control instruction from the main control chip.

24. The nursing machine according to claim 23, wherein the nursing machine further comprises a touch display and a remote controller; the touch display is able to be used for controlling the air pump and/or the relief valve; the touch display is electrically connected to the main control chip; the main control chip is able to transmit a corresponding instruction to the first control chip based on an instruction inputted by the touch display, for the first control chip to control the air pump and/or the relief valve; the remote controller is able to be used for controlling the air pump and/or the relief valve; and the main control chip is able to transmit a corresponding instruction to the first control chip based on an instruction inputted by the remote controller, for the first control chip to control the air pump and/or the relief valve.

25. The nursing machine according to claim 23, wherein the nursing machine further comprises a heater, an air supply apparatus, and a drying pipeline; the at least one control chip further comprises a second control chip; the excrement collection assembly comprises a wrapping pants apparatus and an excrement collector; the excrement collector comprises an excrement recess configured for receiving the human excrement; the air cushion is part of the wrapping pants apparatus; a drying airflow is formed under a heating of the heater and a driving of air by the air supply apparatus; the drying airflow is transmitted through the drying pipeline and supplied to the excrement collector; the second control chip is electrically connected to the main control chip and is able to receive a control instruction from the main control chip; and the heater and the air supply apparatus are respectively electrically connected to the second control chip and controlled by the second control chip.

26. The nursing machine according to claim 25, wherein the at least one control chip further comprises a third control chip; the third control chip is able to receive a control instruction from the main control chip; the negative pressure source is electrically connected to the third control chip and controlled by the third control chip; and the negative pressure source is able to perform suction under control of the third control chip.

27. The nursing machine according to claim 26, wherein the nursing machine further comprises a main unit; the heater and the air supply apparatus are part of the main unit and are located inside a shell of the main unit; the nursing machine comprises a first operating mode and a second operating mode; in the first operating mode, the negative pressure source performs suction under control of the third control chip and produces negative pressure at the excrement recess, and air flows through the air supply apparatus, the heater, and the drying pipeline to the excrement recess of the excrement collector; and in the second operating mode, the negative pressure source stops suction and the heater and the air supply apparatus work under control of the second control chip, to form the drying airflow in the drying pipeline.
